# EUROPÄISCHE PATENTSCHRIFT

(11) **EP 2 634 249 B1**
(45) Veröffentlichungstag und Bekanntmachung des Hinweises auf die Patenterteilung: **17.08.2016**
(21) Anmeldenummer: 12006938.0
(22) Anmeldetag: 07.10.2012
(51) Int. Cl.: C12N 5/00, G01N 33/50

(54) **Verfahren zur Trennung von Verbänden maligner Zellen und Verbänden aus Stromazellen einer ex vivo Malignomgewebeprobe**
Process for the separation of groups of malignant cells and groups of stroma cells from an ex vivo malignant tissue sample
Procédé de séparation d'assemblages de cellules malignes et d'assemblages de cellules de stroma dans un échantillon tissulaire de tumeur maligne ex vivo

(30) Priorität: 28.02.2012 DE 102012003700
(43) Veröffentlichungstag der Anmeldung: 04.09.2013
(73) Patentinhaber: FLACOD GmbH, 69121 Heidelberg (DE)
(72) Erfinder: Granzow, Christof, Prof. Dr., 69121 Heidelberg (DE)
(74) Vertreter: Rudolph, Ulrike

(56) Entgegenhaltungen:
- WO-A2-2004/052184
- US-A1- 2003 096 261
- IRIS-SUSANNE HORN ET AL: "Heterogeneity of epithelial and stromal cells of head and neck squamous cell carcinomas in ex vivo chemoresponse", CANCER CHEMOTHERAPY AND PHARMACOLOGY, SPRINGER, BERLIN, DE, Bd. 65, Nr. 6, 22. September 2009 (2009-09-22), Seiten 1153-1163, XP019800787, ISSN: 1432-0843

## Beschreibung

Die Erfindung betrifft ein Verfahren zur Trennung (Separierung) von Verbänden maligner Zellen und Verbänden von Stromazellen einer Malignomgewebeprobe ex vivo.

Malignome (Synonym: Krebstumore) sind bösartige (maligne ) Tumore, die der normalen Wachstumskontrolle des Organismus entzogen sind, sich ungehindert vermehren, in das umliegende Gewebe eindringen und es zerstören, und die metastasieren können, d.h. sie können in Blut- und Lymphgefäße eindringen und über diese in andere Körperorgane gelangen, wo sie sich wieder ansiedeln und vermehren ( d.h. Metastasen bilden). Demgegenüber zeichnen sich die gutartigen (benignen) Tumore dadurch aus, dass sie nicht invasiv in das umliegende Gewebe einwachsen, sondern es nur verdrängen, und dass sie keine Metastasen bilden.

Den weit überwiegenden Anteil der Malignome bilden die Karzinome. Karzinome sind Malignome, die von Epithelzellen (des Deckepithels der Haut oder des Drüsenepithels der Schleimhäute) ausgehen. Epithelzellen sind normalerweise polarisiert aufgebaut: Sie besitzen basolaterale Seiten, die die Zellen untereinander über interzelluläre Verbindungsstrukturen (z.B. Desmosomen, Tight Junctions, Adherent Junctions) verbinden, eine basale Seite zur extrazellulären Matrix hin, die spezifische Zellmembranproteine und die Basalmembran aufweist, und eine apikale Seite, die mit dem Lumen bzw. der Umwelt in Verbindung steht. Die Integrität der Epithelien wird durch das Bindegewebe unterstützt, in dem sich versorgende Blutgefäße und Fibroblasten befinden. Bei der Entstehung von Karzinomen kommt es zu einer veränderten Zellorganisation des Epithels. Es wird davon ausgegangen, dass die meisten Karzinome durch einzelne entartete Zellen (Vorläuferzellen) hervorgerufen werden, die zu einem Zellklon und schließlich zu einem Tumor heranwachsen. Dieses Wachstum geht mit zahlreichen Veränderungen in der Umgebung der malignen Zellen einher, darunter auch Blutgefäßneubildung (Neoangiogenese), um die Versorgung des Malignoms u.a. mit Nährstoffen und Sauerstoff zu gewährleisten.
Das die malignen Zellen umgebende Gewebe, das sogenannte Tumorstroma, hat sowohl auf zellulärer als auch auf extrazellulärer Ebene einen maßgeblichen Einfluß auf das Malignomwachstum. Dieses Tumorstroma besteht aus Nachbarzellen, insbesondere Endothelzellen und Fibroblasten (Kerbel, 1997), und aus extrazellulärer Matrix, wobei sich sowohl diese Nachbarzellen als auch die Matrix hinsichtlich ihrer Komponenten von den entsprechenden Zellen und der Matrix in gesundem Gewebe unterscheiden.

Obwohl in der Malignom-Therapie (Synonym: Krebs-Therapie) die Behandlung mit Bestrahlung oder mit der Einnahme von Zytostatika oder mit einer Kombination beider Methoden seit Jahrzehnten angewendet wird, beträgt die Erfolgsquote bei den meisten Karzinomen noch immer nur etwa 20 Prozent.
Insbesondere bei der Chemotherapie besteht das Problem, dass eine Behandlung mit einem unpassenden (falschen) oder zu niedrig dosierten Chemotherapeutikum nicht nur keine malignomhemmende Wirkung hat, sondern darüber hinaus zu einer erhöhten Therapieresistenz des Malignoms führen kann. Im Fall von zwei oder mehreren Chemotherapiezyklen mit demselben Chemotherapeutikum, was im Stand der Technik gängige Maßnahme ist, sind die Erfolgsraten nach der zweiten und weiteren Behandlung folglich in der Regel noch viel niedriger, als nach der ersten Behandlung.
Gegenwärtig sind die allermeisten Behandlungspläne in der Chemo- und Strahlentherapie mehr oder weniger empirisch definiert, was schon deshalb problematisch ist, weil jedes Malignom eines Individuums auf ein Chemotherapeutikum und/oder eine Bestrahlung individuell reagiert.

Für eine effektive und auf das spezifische Malignom eines Individuums ausgerichtete/zugeschnittene Therapie wurden aber auch schon Testverfahren vorgeschlagen, mit denen vor Verabreichung einer geplanten Chemo- und/oder Strahlentherapie die Sensitivität der Malignomzellen auf das geplante Therapeutikum, insbesondere das Chemotherapeutikum, geprüft werden kann. Die Durchführung eines solchen Testverfahrens erfolgt ex vivo an einer frisch isolierten Malignomgewebeprobe. Der Begriff "ex vivo" bezeichnet (im Kontext vorliegender Beschreibung) eine Zellkulturtechnik, bei der frisch aus dem Organismus entnommene Gewebeproben verwendet werden. (Aus solchen Gewebeproben werden beispielsweise die sogenannten Primär(-zell-)kulturen hergestellt, die in der Regel zellulär inhomogen sind.) Diese Malignomgewebeprobe wird zunächst zerkleinert und mehr oder weniger vollständig in Einzelzellen aufgelöst, dann mit dem fraglichen Therapeutikum inkubiert und anschließend das Wachstumsverhalten der so behandelten Zellen bestimmt. Durch Inkubation mit verschiedenen Konzentrationen des Therapeutikums kann dessen IC50-Wert bestimmt werden. Der IC50-Wert eines Chemotherapeutikum steht für diejenige Konzentration dieses Mittels, bei der die Proliferation der damit behandelten und untersuchten Zellen im Vergleich zu unbehandelten Kontrollzellen auf 50% reduziert ist. Er ist ein wichtiges Kriterium für die Beschreibung der Malignomreaktion auf ein Chemotherapeutikum. Anhand der Testergebnisse lässt sich vorhersagen, wie stark empfindlich das Malignom für das Therapeutikum ist und wie der Behandlungsplan bezüglich Dosis und Verabreichungszyklen infolgedessen gewählt werden sollte, um den gewünschten Malignom-hemmenden oder Malignom-vernichtenden Effekt zu erreichen. Solche Testverfahren sind z.B. in der US 2001/0051353 A1 und in der WO 2009/124997 A1 beschrieben.

Bezüglich der zellulären Chemoresponse (d.h. der zellulären Reaktion auf ein bestimmtes Chemotherapeutikum) ist geltende Lehrmeinung, dass nur die malignen Zellen eines Malignoms zur Resistenzentwicklung befähigt sind, nicht dagegen die Zellen des Malignomstromas (Kerbel, 1997). Während der letzten Jahre wurde jedoch auch in Stromazellen von Malignomen eine Chemoresistenz nachgewiesen, so z.B. in primären und metastatischen Lungenkarzinomen (Granzow et al., 2004) und in Kopf-Hals-Karzinomen (Dollner et al., 2004). Bei beiden genannten Karzinomentitäten wurden sowohl Malignome mit chemoresistenten malignen Zellen und chemoresistenten Stromazellen nachgewiesen, als auch Malignome mit chemosensiblen malignen Zellen und chemosensiblen Stromazellen, als auch Malignome mit chemosensiblen malignen Zellen und chemoresistenten Stromazellen, und ebenso Malignome mit chemoresistenten malignen Zellen und chemosensiblen Stromazellen. Die Identifizierung und Untersuchung der diversen Zelltypen erfolgte bei diesen Nachweisen jeweils unter Verwendung von Proben menschlicher Malignome ex vivo in geeigneten Zellkulturschalen, wobei die erwähnten Zelltypen in Gegenwart oder Abwesenheit von Zytostatika ko-kultiviert wurden (Dietz et al, 2009).
Aus dieser Feststellung, dass in einem Malignom Stromazellen und maligne Zellen unabhängig voneinander deutlich verschiedene Sensibilitäten für ein bestimmtes Therapeutikum aufweisen können, folgt, dass die vorstehend beschriebenen bekannten Testverfahren zur Überprüfung von Malignomzellen einer Malignomgewebeprobe (von einem Patienten) hinsichtlich ihrer Sensitivität für ein zur Verabreichung geplantes Therapeutikums, insbesondere ein Chemotherapeutikum, nicht selten falsch-negative oder falsch-positive Ergebnisse liefern.
Auch das Ergebnis einer molekularen Analyse des Genexpressionsprofils hinsichtlich Chemoresistenzgenen (analysis of molecular tumor chemoresponse signatures), die mit einer solchen Malignomgewebeprobe durchgeführt wird, kann deshalb verfälscht sein, weil chemosensitive Stromazellen mit chemoresistenten malignen Zellen koexistieren können.

Aus der US 2003/096261 ist ein Verfahren zur Gewinnung von Endothelzellen aus Tumorgewebe bekannt, bei dem angestrebt wird, dass die Endothelzellen am Ende als pure Einzelzell-Suspension (""near single cell suspension") vorliegen. Auch die WO 2004/052184 A2 beschreibt ein Verfahren zur Überführung/Aufschließung einer Tumorgewebeprobe in eine pure Einzelzell-Suspension ("near single cell suspension"). In beiden Schriften wird zur Gewinnung der angestrebten Einzelzell-Suspension Collagenase und DNase eingesetzt, zusätzlich zu dem enzymatischen Verdau wird eine mechanische Einwirkung in Form von Rühren durchgeführt, und das Verdauprodukt wird unidirektional durch einen Filter passiert.

Die Publikation von Iris-Susanne Horn et al., 2010 ("Heterogenneity of epithelial and stromal cells of head and neck squamous cell carcinomas in ex vivo chemoresponse" Cancer Chemother Pharmacol (2010) 65: 1153-1163, Springer-Verlag Berlin) betrifft ein Testverfahren, mit dem vor Verabreichung einer geplanten Chemo- und/oder Strahlentherapie die Sensitivität der Malignomzellen auf das geplante Therapeutikum, insbesondere das Chemotherapeutikum, prüfbar sein soll. Für die Durchführung dieses Testverfahrens werden die eingesetzten Zellen aus der betreffenden, frisch isolierten Malignomgewebeprobe dadurch gewonnen, dass ein "collagenase digest" hergestellt wird. Mit diesem "collagenase digest", in dem maligne Zellen (Tumor-Epithelzellen) und Stromazellen nebeneinander vorliegen, werden anschließend die Chemoresponse-Tests durchgeführt. Erst nach erfolgter Inkubation in Anwesenheit des Chemotherapeutikums werden die Zellen fixiert und gefärbt und dadurch identifizierbar und unterscheidbar gemacht.

Der vorliegenden Erfindung liegt die Aufgabe zugrunde, diese Nachteile des Stands der Technik zu beheben oder wenigstens zu mindern.

Eine Lösung dieser Aufgabe besteht in der Bereitstellung eines Verfahren zur Trennung (Separierung und Abtrennung) von Verbänden aus malignen Zellen und Verbänden aus Stromazellen (insbesondere Endothelzellen und Fibroblasten) einer ex vivo Malignomgewebeprobe, das durch Art und Reihenfolge der folgenden Schritte gekennzeichnet ist:
(a) Die (vorzugsweise frisch gewonnene) Tumorgewebeprobe wird zunächst (vorzugsweise binnen maximal 9 Minuten) von nekrotischen Anteilen und anhängendem sonstigem (Nicht-Malignom-) Gewebe gereinigt und zerkleinert (vorzugsweise zerschnitten), vorzugsweise in Stücke von etwa 1 bis 4 mm³ (Kubikmillimeter) Volumen.
(b) Das zerkleinerte Gewebe wird in (vorzugsweise Serum-haltigem) Kulturmedium aufgeschwemmt (suspendiert).
(c) Die gewonnene Suspension wird einer Behandlung mit Kollagenase (d.h. einem Kollagenaseverdau bzw. einem Kollagenaseaufschluß) für einen partiellen Kollagenaseverdau unterworfen, wobei *Clostridium histolyticum* Kollagenase in einer Konzentration von 200 CDU bis 250 CDU pro ml bei etwa 36°C bis 37°C für die Dauer von etwa 4 bis 5 Stunden eingesetzt wird.
(d) Das dadurch erhaltene Kollagenaseaufschlußprodukt wird zentrifugiert, vorzugsweise für die Dauer von etwa 5 Minuten bei 50 x g und Raumtemperatur.
(e) Das gewonnene Pellet wird vom Überstand abgetrennt.
(f) Das am Ende von Schritt (e) vorliegende Pellet des Kollagenaseverdauprodukts aus (e) wird mittels eines Aufsauginstruments (beispielsweise einer Pipettenspitze) in Kulturmedium aufgeschlossen und resuspendiert, dabei etwa 4-6 mal rasch aufgenommen, zurückgegeben (zurück fließen gelassen) und wieder eingesaugt und damit einer Behandlung mit hydrodynamischen Scherkräften unterworfen, und anschließend in ein Kulturgefäß überführt.
(g) Die in (f) gewonnene, durch mehrfaches aufsaugen und wieder abgeben behandelte Resuspension wird mit Hilfe eines Mikroskops (vorzugsweise eines Inversmikroskops mit Phasenkontrastoptik) hinsichtlich wenigstens eines Zellverbands analysiert, der entweder aufgrund seines phänotypischen Erscheinungsbildes als Verband aus malignen Zellen identifizierbar ist, oder der aufgrund seines phänotypischen Erscheinungsbildes als Verband aus Endothelzellen identifizierbar ist, oder der aufgrund seines phänotypischen Erscheinungsbildes als Verband aus Fibroblasten identifizierbar ist.
Die Zellverbände von/aus malignen Zellen sind phänotypisch gekennzeichnet durch eine annähernd runde Form des Zellverbands, durch Zellen mit einem relativ großen Durchmesser und einem hohen Phasenkontrast und durch eine kompakte Zusammenlagerung der Zellen in diesem Zellverband. Demgegenüber sind die Verbände von Stromazellen im Fall von Endothelzellen phänotypisch gekennzeichnet durch Zellen mit einer augenscheinlich geringeren Größe und einem geringeren Phasenkonstrast im Vergleich zu den malignen Zellen im Zellverband und einer dicht gepackten Zusammenlagerung in Form gewundener, länglicher Röhren. Im Fall von Fibroblasten sind die Verbände von Stromazellen phänotypisch gekennzeichnet durch eine geringere Größe im Vergleich zu den malignen Zellen im Zellverband und durch eine enge Assoziation mit teilverdauten Kollagenfaserbündeln. (h) Die in (g) indentifizierten Zellverbände werden separiert, beispielsweise durch Überführung in separate Kultur- oder Testgefäße.

Hier und im folgenden steht der Begriff "maligne Zellen" für diejenigen Zellen eines Malignoms, die autonom proliferieren, die zum invasiven-destruktiven Wachstum fähig sind und die Metastasen bilden können.
Der Begriff "Stromazellen" steht hier und im folgenden zum einen für diejenigen Zellen eines Malignoms, die mit den malignen Zellen ko-proliferieren, jedoch keine biologische Malignität aufweisen. Bei diesen Stromazellen handelt es sich vor allem um Fibroblasten und zelluläre Komponenten des Blutgefäßsystems, insbesondere Endothelzellen. Zum anderen umfasst der Begriff "Stromazellen" aber auch alle nicht-malignen Zellen in einem Malignom, die ex vivo nicht proliferationsfähig sind.
Der Begriff "Zellverband" (Plural: "Zellverbände") steht hier und im folgenden für im Gewebe eines Malignoms vorbestehende (d.h. ursprünglich bereits bestehende), infolge der Einwirkung von Kollagenase und Scherkräften (beim Ansaugen und wieder Abgeben in das/aus dem Ansauginstrument) freigesetzte, zellulär weitgehend homogene Aggregationen von Zellen, z.B. von malignene Zellen, von Fibroblasten oder von Endothelzellen.
Die Abkürzung "*g*" steht für die mittlere Erdbeschleunigung.

Mit dem erfindungsgemäßen Verfahren gehen die Vorteile einher, dass der partielle Kollagenaseverdau gefolgt von einer kurzen, milden hydromechanischen Behandlung (mit Scherkräften) zur Bildung von vereinzelten Zellverbänden führt, die entweder aus malignen Zellen oder aus einem der Stromazelltypen Fibroblasten oder Endothelzellen bestehen, und die einfach und leicht anhand ihres phänotypischen Erscheinungsbildes identifiziert und separiert werden können. Insbesondere die malignen Zellen sind weitgehend in ihrem ursprünglichen Zustand (d.h. dem Zustand im Malignom in-vivo) erhalten. Im Fall von Karzinomazellen beispielsweise bestehen die Zellverbände aus phänotypisch zusammengehörenden, aufeinander abgestimmten Einzelzellen, die über Zellverbindungsstrukturen (z.B. Desmosomen) miteinander in Kontakt stehen.

So können auf schnelle, kostengünstige und technisch unaufwendige Art und Weise aus der ursprünglich gewonnenen Malignomgewebeprobe die malignen Zellen isoliert werden und nur diese malignen Zellen einem Testverfahren zur Überprüfung ihrer Sensitivität für ein bestimmtes zur Anwendung geplantes Chemo- oder Strahlentherapeutikum unterworfen werden.

Die frisch gewonnene Malignomgewebeprobe kann insbesondere im Zuge einer Resektion oder Biopsie erhalten worden sein.

Bei dem erfindungsgemäßen Verfahren wird als Kollagenase *Clostridium histolyticum* Kollagenase für die Dauer von etwa 4 bis 5 Stunden eingesetzt, weil damit ein relativ milder, nur partieller Kollagenaseverdau durchgeführt werden kann. Vorzugsweise sollte die Dauer von 5 Stunden nicht überschritten werden. Die eingesetzte Menge an Kollagenase beträgt etwa 200 CDU bis 250 CDU pro ml, wobei insbesondere mit etwa 230 CDU/ml sehr gute Ergebnisse erhalten werden können.

In Schritt (h) des Verfahrens kann die Resuspension sowohl hinsichtlich Zellverbänden von malignen Zellen als auch hinsichtlich Zellverbänden von Fibroblasten und/oder Zellverbänden von Endothelzellen analysiert werden, und die identifizierten Zellverbände können isoliert werden, z.B. indem sie mit Hilfe eines Aufsauginstruments separat (einzeln) aufgenommen und jeweils in ein separates Kulturgefäß überführt werden. Als Aufsauginstrument kommt hier insbesondere eine Injektionskanüle Nr. 23 (Durchmesser 0,6 mm, Länge 60-80 mm) aufgesteckt auf eine 2-ml-Injektionspritze in Betracht.

Falls gewünscht, können auch nur identifizierte Zellverbände von Endothelzellen und/oder nur identifizierte Zellverbände von Fibroblasten aus der Resuspension entnommen werden - z.B. weil diese weitergehenden Untersuchungen unterworfen werden sollen. Als eine solche weitergehende Untersuchung kommt erfindungsgemäß insbesondere ein Verfahren zur Überprüfung der Wirkung von Hemmstoffen der Angioneogenese auf die separierten Zellverbände von Endothelzellen in Betracht.

Es können aber auch alle identifizierten Zellverbände von Endothelzellen und alle Zellverbände von Fibroblasten aus der Resuspension entnommen werden, so daß nur noch maligne Zellen zurückbleiben. Die so gewonnene zellulär homogene oder zumindest weitgehend homogene Suspension aus malignen Zellen in Kulturmedium steht für weiterführende Untersuchungen an den malignen Zellen zur Verfügung und hat den Vorteil, dass die malignen Zellen nur einmal der Saugbehandlung und den damit einhergehenden hydrodynamischen (Scher-)kräften ausgesetzt waren.

In einer Variante des erfindungsgemäßen Verfahrens erfolgt in Schritt (g) die Trennung der in der Resuspension enthaltenen verschiedenen Zellverbände im Zuge einer Dichtegradientenzentrifugation anhand ihres unterschiedlichen Sedimentationsverhaltens. Zu diesem Zweck wird die in (f) gewonnene Resuspension in eine Trennlösung aus Saccharose-Polymer in Kulturmedium, unterworfen. Als Saccharose-Polymer wird vorzugsweise Saccharose-Epichlorhydrin-Copolymer (z.B. Ficoll ®) eingesetzt. Im verlauf dieser Dichtegradientenzentrifugation sedimentieren die Verbände maligner Zellen bei höheren Konzentrationen des Saccharose-Polymers, insbesondere des Saccharose-Epichlorhydrin-Copolymers, als die Verbände von Fibroblasten oder die Verbände von Endothelzellen.

Anstelle der Dichtegradientenzentrifugation kann ebenso gut auch eine Zentrifugal-Elutriation durchgeführt werden. Zu diesem Zweck wird die in (f) gewonnene Resuspension in eine im Stand der Technik bekannte Trennkammer für Zentrifugal-Elutriation überführt und anschließend elutriiert. Hierbei sammeln sich die Verbände aus/von malignen Zellen bei höheren Durchflussgeschwindigkeiten als die Verbände aus/von Stromazellen an.

Das erfindungsgemäße Verfahren ist erfindungsgemäß insbesondere auch als Vorverfahren für ein Verfahren zur Evaluierung/Überprüfung der Antwort von malignen Zellen einer Malignomgewebeprobe ex vivo auf ein bekanntes Chemotherapeutikum und/oder Strahlentherapeutikum vorgesehen. Mit anderen Worten: das erfindungsgemäße Verfahren ist insbesondere dafür vorgesehen, Malignomgewebeproben ex vivo für ein Verfahren zur Überprüfung ihrer Sensibilität und möglichen Resistenz auf ein bekanntes Chemotherapeutikum und/oder Strahlentherapeutikum vorzubereiten (bzw. vorzubehandeln bzw. zu konditionieren).
Bei dem Verfahren zur Überprüfung von Sensibilität und möglichen Resistenz auf ein bekanntes Chemotherapeutikum und/oder Strahlentherapeutikum kann es sich sowohl um einen Zellwachstumstest handeln, bei dem die Zellen mit dem Chemotherapeutikum und/oder Strahlentherapeutikum inkubiert bzw. behandelt werden (im Stand der Technik bekannt z.B. aus der US 2001/0051353 A1 und der WO 2009/124997 A1), oder auch um ein Verfahren zur molekularen Analyse des Genexpressionsprofils. Solche Verfahren zur molekularen Analyse des Genexpressionsprofils sind im Stand der Technik bekannt, z.B. aus Potti et al. 2006

Gegenstand der Erfindung ist deshalb auch ein Verfahren zur Evaluierung/Überprüfung der wachstumshemmenden Wirkung eines bekannten Chemotherapeutikum und/oder Strahlentherapeutikum auf eine Malignomgewebeprobe ex vivo, bei dem das Chemotherapeutikum und/oder Strahlentherapeutikum mit der Malignomgewebeprobe inkubiert und anschließend die Anzahl der Zellen und/oder Zellkolonien analysiert und schließlich der IC50-Wert von Chemotherapeutikum oder Strahlentherapeutikum oder der Kombination von Chemo- und Strahlentherapeutikum bestimmte wird, und das sich dadurch auszeichnet, dass die Malignomgewebeprobe vor Inkubation mit dem Chemotherapeutikum und/oder Strahlentherapeutikum in maligne Zellen und Stromazellen aufgeschlossen wird und nur maligne Zellen mit dem Chemotherapeutikum und/oder Strahlentherapeutikum inkubiert hinsichtlich des C50-Wertes analysiert werden.
Der Begriff Strahlentherapeutikum steht im vorliegenden Kontext insbesondere für isonisierende Strahlung.

Mit anderen Worte: erfindungsgemäß wird vorgeschlagen, die ex vivo Malignomgewebeprobe zuerst einem Verfahren zur Trennung (Separierung und Abtrennung) von malignen Zellen und Stromazellen zu unterwerfen, vorzugsweise dem erfindungsgemäßen Verfahren, und anschließend nur die separierten und abgetrennten malignen Zellen in einem Verfahren zur molekularen Analyse des Genexpressionsprofils und/oder in einem Verfahren zu Evaluierung/Überprüfung auf Sensitivität bzw. Resistenz gegen über einem gewählten mutmaßlichen Chemotherapeutikum und/oder Strahlentherapeutikum einzusetzen.

Bei dem Verfahren zur Evaluierung/Überprüfung einer Malignomgewebeprobe auf Sensitivität bzw. Resistenz gegenüber einem bekannten mutmaßlichen Chemotherapeutikum und/oder Strahlentherapeutikum kann es sich insbesondere um ein ex vivo Verfahren handeln, das die folgenden Maßnahmen umfasst:
- Eine frisch isolierte Malignomgewebeprobe wird in ein Behältnis überführt, und in Gegenwart von Antibiotika und vorzugsweise auch Antimykotika bei einer Temperatur von über 10°C, vorzugsweise über 15°C gehalten.
- Die Malignomgewebeprobe wird, vorzugsweise mechanisch, in kleine Stücke zerteilt.
- Die Malignomgewebeprobe-Stücke werden innerhalb von maximal 12 Stunden, vorzugsweise binnen 5 Stunden nach Beginn des Verfahrens in Zellkulturmedium mit einem Gehalt an Kollagenase überführt und mindestens 10 Stunden darin inkubiert, anschließend gewaschen und danach in Zellkulturmedium resuspendiert.

- Die dadurch gewonnenen Zellen und Gewebeteile werden in Behältnisse mit einer Beschichtung aus extrazellulären Matrix-Komponenten überführt, auf dieser Beschichtung ausplattiert und mit dem Chemotherapeutikum und/oder Strahlentherapeutikum inkubiert.
- Anschließend wird die Anzahl der Zellen und/oder die Anzahl der Kolonien analysiert, vorzugsweise nach Durchführung einer Cytokeratin-Färbung.
- Das verwendete Zellkulturmedium enthält weniger als 100 nmol pro 1 Liter Flavin und ist frei von Phenol rot.
- Alle Maßnahmen im Anschluß an die Kollagenasebehandlung werden in Abwesenheit von Licht einer Wellenlänge unter 520 nm durchgeführt.
- Der IC50-Wert für das Chemo- oder Strahlentherapeutikum oder für die Kombination aus Chemo- und Strahlentherapeutikum wird bestimmt.

Wird dieses Verfahren zur Überprüfung einer Malignomgewebeprobe auf Sensitivität oder Resistenz gegen über einem bekannten mutmaßlichen Chemotherapeutikum und/oder Strahlentherapeutikum mit dem erfindungsgemäßen Verfahren zur Trennung von malignen Zellen und Stromazellen kombiniert/gekoppelt, dann ersetzt das erfindungsgemäße Trennungsverfahren die genannten ersten (drei) Schritte bis einschließlich der Kollagenasebehandlung dieses Überprüfungsverfahrens.

Die Erfindung wird im folgenden anhand von Ausführungsbeispielen und dazugehörigen Figuren näher erläutert:

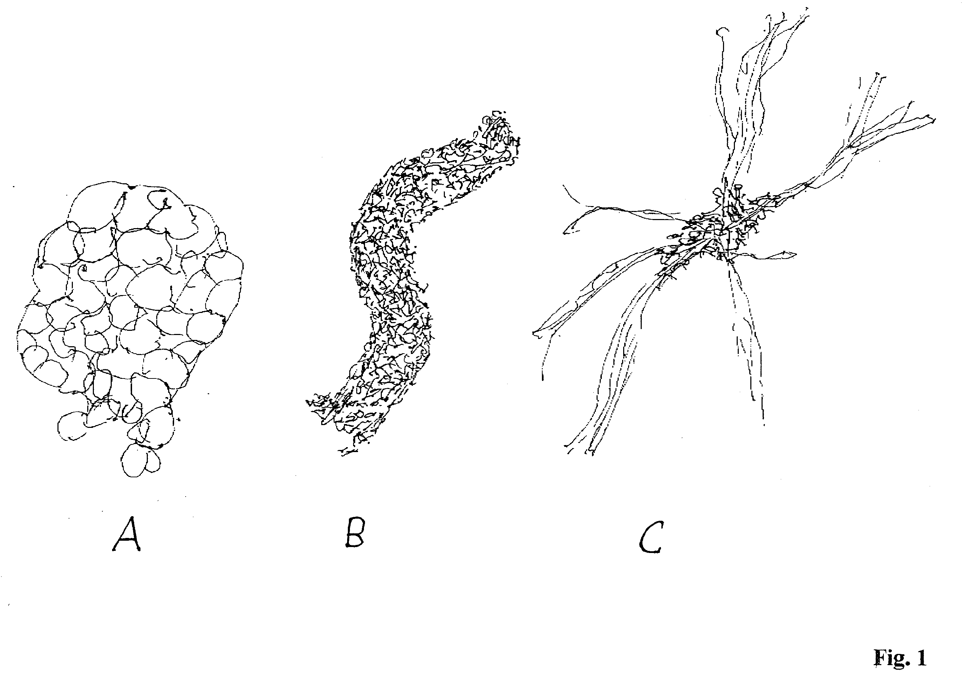

- Figur 1 zeigt:: die schematische Wiedergabe des phasenoptischen Bildes eines Verbands maligner Zellen (A), eines Verbands von Endothelzellen (B) und eines Verbands von faser-assoziierten Fibroblasten (C), jeweils erhalten mit dem erfindungsgemäßen Verfahren.

### Beispiel 1: Trennung von malignen Zellen und Stromazellen bei Gewebeproben von Lungenmalignomen

Von Lungenmalignomen mit unterschiedlicher Histologie wurden Proben frisch resektiert, gereinigt und klein geschnitten. Fünf bis 10 mg Tumorprobengewebe pro 1 ml wurden in RPMI 1640 Kulturmedium mit einem Gehalt von 10 % FBS resuspendiert. Diese Suspensionen wurden einem Kollagenaseverdau unterworfen, vorzugsweise unter Einsatz von Clostridium histolyticum Kollagenase (230 CDU/ml) für die Dauer von 4 bis 5 Stunden bei 36°C bis 37°C, vorzugsweise bei 36,5 °C. Anschließend wurde das Aufschlußprodukt bei 50 x g zentrifugiert, der Überstand verworfen und das Pellet in 1 ml frischem Kulturmedium aufgeschlossen und resuspendiert. Jeder Ansatz wurde in eine Petrischale überführt und mit Medium auf eine Konzentration entsprechend 5 bis 10 mg Tumorproben-Feuchtgewicht pro 1 ml Kulturmedium eingestellt.

Die Zellsuspension jedes Ansatzes wurde mit einer 1 ml-Pipettenspitze (ein anderes Aufsauginstrument kann ebenso verwendet werden) 4 bis 5 mal hintereinander aufgenommen und wieder ausgegeben und damit einer Behandlung mit hydrodynamischen Scherkräften unterworfen. Anschließend wurden mit Hilfe eines Inversmikroskops und Phasenkonstrastoptik Zellverbände phänotypisch gleichartiger Zellen detektiert, und jeweils mit auf 2 ml-Injektionsspritzen aufgesetzten Injektionskanülen Nr. 23 (Durchmesser 0,6 mm, Länge 60-80 mm) in ein separates Kulturgefäß überführt.

Die Reinheit der auf diese Weise gewonnenen Zellverbände wurde mittels Phasenkontrastmikroskopie überprüft.

Ansätze dieser Zellverbände sowie der unfraktionierten Aufschlußprodukte wurden hinsichtlich ihrer Chemosensitivität gegenüber Cisplatin untersucht. Dafür wurden die Ansätze in mit extrazellulärer Matrix (ECM) beschichteten Kulturplatten in Gegenwart von 0 nM, 60 nM, 600 nM und 6000 nM Cisplatin für 3 Tage inkubiert. Anschließend wurden die Zellen mit Methanol fixiert, einer Giemsafärbung oder einer Immunfärbung mit einem Anti-Zytokeratin-Antikörper (beispielsweise dem Antikörper C11) unterworfen und schließlich mikroskopisch analysiert.

Die mikroskopisch-phasenoptische Analyse der Aufschlussprodukte ergab, dass in ihnen ko-existierende, beständige Verbände aus räumlich eng assoziierten Zellen unterschieden werden können, bei denen es sich entweder um maligne Zellen oder um Endothelzellen oder um faser-assoziierte Fibroblasten handelt. Verbände aus malignen Zellen erscheinen unter dem Phasenkontrastmikroskop rund oder rundlich. Ihre Zellen haben relativ große Durchmesser und hohen Phasenkontrast und liegen dicht gepackt (vgl. Fig. 1 A). Solche Zellverbände sedimentieren schnell. Verbände aus Endothelzellen bilden gewundene Röhren, die an Blutgefäße erinnern. Die Endothelzellen selbst erscheinen im Vergleich zu den malignen Zellen kleiner, weisen einen geringeren Phasenkonstrast auf und liegen dicht gepackt (vgl. Fig. 1B ). Verbände aus Fibroblasten liegen oft in enger Nachbarschaft zu oder angelagert an enzymatisch fragmentierte Kollagenbündel. Die Fibroblasten selbst erscheinen kleiner als die malignen Zellen (vgl. Fig. 1C). Die Verbände aus Endothelzellen und ebenso die Verbände aus Fibroblasten sedimentieren signifikant langsamer als die Verbände aus malignen Zellen.
Mit Hilfe von im Stand der Technik bekannten und geläufigen immunhistochemischen Methoden kann der Fachmann nach (beispielsweise dreitägiger) Inkubation in ECM-Platten und Fixierung) die zelluläre Homogenität oder zumindest Beinahe-Homogenität sowohl bei den entstandenen Kolonien von malignen Zellen als auch bei solchen der Stromazellen, d.h. der Endothelzellen und der Fibroblasten, zuverlässig oder zumindest annähernd zuverlässig feststellen.
Die meisten der unfraktionierten und der fraktionierten Aufschlussprodukte enthalten Verbände von malignen Zellen und/oder Stromazell-Verbände, die auch in Gegenwart von 6000 nM Cisplatin zur Koloniebildung ex vivo fähig sind.

Mit diesem Beispiel wird zum einen gezeigt, dass ein partieller Kollagenaseverdau von Lungenmalignomgewebe, gefolgt von einer kurzen, milden hydromechanischen Behandlung, zur Freisetzung von prä-existenten Zellverbänden führt, die entweder aus malignen Zellen oder aus verschieden Stromazelltypen bestehen, und die einfach und leicht separiert werden können.

Die gewonnenen Ergebnisse im Sensitivitätstest mit Cisplatin zeigen außerdem, dass die isolierten, mikroskopisch identifizierten Stromazellverbände auch in Abwesenheit von malignen Zellen häufig eine Cisplatin-Resistenz aufweisen, die auf einer entsprechenden Genexpression beruhen dürfte.

### Zitierte Literatur:

Dietz A, Tschöp K, Wichmann G, Granzow C: Method and kit for the ex vivo evaluation of the response of a tumor to conditions to be tested. International Publication Number WO 2009/124997 A1
Dollner R, Granzow C, Helmke BM, Ruess A, Schad A, Dietz A: The impact of stromal cell contamination on chemosensitivity testing of head and neck carcinoma. Anticancer Res. 24:325-31 (2004)
Granzow C, Kopun M, Heuser M, Herth F, Becker HD: Chemoresistance of human lung tumor stromal cells. Amer. Assn. Cancer Res. 95th Annual Meeting Proc. Suppl., abstract LB-82 (2004)
Kerbel R S: A cancer therapy resistant to resistance. Nature 390: 335-6 (1997)
Potti A, Dressman HK, Bild A, Riedel RF, Chan G, Sayer R, Cragun J, Cottrill H, Kelley MJ, Petersen R, Harpole D, Marks J, Berchuck A, Ginsburg GS, Febbo P, Lancaster J, Nevins JR.: Genomic signatures to guide the use of chemotherapeutics. Nat. Med. 12:1294-1300 (2006)

## Patentansprüche

1. Verfahren zur Trennung von Verbänden maligner Zellen und Verbänden aus Stromazellen einer Malignomgewebeprobe ex vivo, **gekennzeichnet durch** Art und Reihenfolge der folgenden Schritte:
(a) die Malignomgewebeprobe wird gereinigt und zerkleinert, vorzugsweise in Stücke von etwa 1-4 mm³ (Kubikmillimeter) Volumen,
(b) das zerkleinerte Gewebe wird in Kulturmedium suspendiert
(c) die in (b) gewonnene Suspension wird einer ausschließlichen Behandlung mit ausschließlich Kollagenase (d.h. einem Kollagenaseverdau bzw. einem Kollagenaseaufschluß ) für einen partiellen Kollagenaseverdau unterworfen, wobei *Clostridium histolyticum* Kollagenase in einer Konzentration von 200 CDU bis 250 CDU pro ml bei etwa 36°C bis 37°C für die Dauer von etwa 4 bis 5 Stunden, eingesetzt wird;
(d) das in (c) gewonnenen Kollagenaseverdauprodukt wird zentrifugiert, vorzugsweise etwa 5 Minuten bei 50 x g und Raumtemperatur,
(e) das in (d) gewonnene Pellet wird vom Überstand abgetrennt,
(f) das Pellet des Kollagenaseverdauprodukts aus (e) wird in Kulturmedium aufgeschlossen und resuspendiert und dabei mittels eines Aufsauginstruments, vorzugsweise einer Pipettenspitze, etwa 4-6 mal aufgenommen und wieder zurückgegeben und wieder eingesaugt und damit einer Behandlung mit hydrodynamischen Scherkräften unterworfen, und anschließend in ein Kulturgefäß überführt,
(g) die in (f) gewonnene Resuspension wird mit Hilfe eines Mikroskops (vorzugsweise eines Inversmikroskops mit Phasenoptik) hinsichtlich wenigstens eines Zellverbands analysiert, der entweder aufgrund seines phänotypischen Erscheinungsbildes als Verband maligner Zellen identifizierbar ist, oder aufgrund seines phänotypischen Erscheinungsbildes als Verband von Endothelzellen identifizierbar ist, oder aufgrund seines phänotypischen Erscheinungsbildes als Verband von Fibroblasten identifizierbar ist, und
(h) die in (g) identifizierten Zellverbände werden separiert.

2. Verfahren nach Anspruch 1, **dadurch gekennzeichnet, dass** in Schritt (c) die *Clostridium histolyticum* Kollagenase in einer Konzentration von etwa 230 CDU/ml eingesetzt wird.

3. Verfahren nach Anspruch 1 oder 2, **dadurch gekennzeichnet, dass** in Schritt (g) die Resuspension sowohl hinsichtlich Zellverbänden von malignen Zellen als auch hinsichtlich Zellverbänden von Fibroblasten und/oder Zellverbänden von Endothelzellen analysiert wird, und dass die identifizierten Zellverbände separat aufgenommen werden, vorzugsweise mittels eines Aufsauginstruments, und jeweils in ein separates Kulturgefäß überführt werden.

4. Verfahren nach einem der Ansprüche 1 bis 3, **dadurch gekennzeichnet, dass** in Schritt (g) die Resuspension einer Dichtegradientenzentrifugation in einer Trennlösung aus Saccharose-Polymer, vorzugsweise Saccharose-Epichlorhydrin-Copolymer (z.B. Ficoll), in Kulturmedium unterworfen wird, wobei die Verbände maligner Zellen bei höheren Konzentrationen des Saccharose-Polymers sedimentieren als die Verbände von Fibroblasten oder Endothelzellen.

5. Verfahren nach einem der Ansprüche 1 bis 3, **dadurch gekennzeichnet, dass** in Schritt (g) die Resuspension in eine Trennkammer für Zentrifugal-Elutriation überführt und anschließend elutriiert wird, wobei sich die Verbände maligner Zellen bei höheren Durchflussgeschwindigkeiten ansammeln als die Verbände von Fibrobasten oder die Verbände von Endothelzellen.

6. Verfahren nach einem der Ansprüche 1 bis 5 **dadurch gekennzeichnet, dass** es ein Vorverfahren für ein Verfahren zur Überprüfung der Sensitivität und möglichen Resistenz der Zellen einer Malignomgewebeprobe ex vivo auf ein bekanntes Chemotherapeutikum und/oder Strahlentherapeutikum ist.

7. Verfahren zur Überprüfung der Antwort von malignen Zellen einer Malignomgewebeprobe ex vivo auf ein bekanntes Chemotherapeutikum und/oder Strahlentherapeutikum, **dadurch gekennzeichnet,**
**dass** die Malignomgewebeprobe zuerst einem Verfahren gemäß einem der Ansprüche 1 bis 5 unterworfen wird, und dass nur die dabei identifizierten Verbände maligner Zellen in dem Verfahren zur Evaluierung/Überprüfung eingesetzt werden.

8. Verfahren nach Anspruch 7, **dadurch gekennzeichnet, dass** das Verfahren zur Überprüfung folgende Maßnahmen aufweist:
i) Überführung der identifizierten Verbände maligner Zellen in Behältnisse mit einer Beschichtung aus extrazellulären Matrix-Komponenten, Ausplattierung auf dieser Beschichtung und Inkubierung mit dem Chemotherapeutikum und/oder Strahlentherapeutikum,
ii) Analysierung der Anzahl der Zellen und/oder Anzahl der Kolonien, vorzugsweise nach Durchführung einer Cytokeratin-Färbung,
iii) wobei das in den Schritten i) und ii) verwendete Zellkulturmedium weniger als 100 nmol pro 1 Liter Flavin enthält und frei ist von Phenol rot,
iv) und wobei die Schritte i) und ii) in Abwesenheit von Licht einer Wellenlänge unter 520 nm durchgeführt werden,
v) Bestimmung des IC50-Wertes für das Chemo- oder Strahlentherapeutikum oder für die Kombination aus Chemo- und Strahlentherapeutikum.

9. Verfahren zur Überprüfung der wachstumshemmenden Wirkung eines bekannten Chemotherapeutikums und/oder Strahlentherapeutikums auf eine Malignomgewebeprobe ex-vivo, bei dem das Chemotherapeutikum und/oder Strahlentherapeutikum mit der Malignomgewebeprobe inkubiert und anschließend die Anzahl der Zellen und/oder Zellkolonien analysiert und letztlich der IC50-Wert des Chemotherapeutikums oder Strahlentherapeutikums oder der Kombination von Chemo- und Strahlentherapeutikum bestimmt wird, und das sich dadurch auszeichnet, dass die Malignomgewebeprobe vor Inkubation mit dem Chemotherapeutikum und/oder Strahlentherapeutikum mit einen Verfahren nach einem der Ansprüche 1 bis 5 in Stromazellen und maligne Zellen aufgeschlossen wird, und dass nur maligne Zellen mit dem Chemotherapeutikum und/oder Strahlentherapeutikum inkubiert und anschließend hinsichtlich ihres IC50-Wertes analysiert werden

10. Verfahren nach einem der Ansprüche 3 bis 5, **dadurch gekennzeichnet, dass** die separierten Zellverbände von Endothelzellen einem Verfahren zur Überprüfung der Wirkung von Hemmstoffen der Angioneogenese unterworfen werden.

## Claims

1. A method for separating clusters of malignant cells and clusters of stromal cells of a malignant tumor tissue sample *ex vivo,* **characterized by** manner and order of the following steps:
(a) the malignant tumor tissue sample is purified and minced, preferably in pieces of about 1-4 mm³ (cubic millimetre) volume,
(b) the minced tissue is suspended in culture medium,
(c) the suspension obtained in (b) is subjected to an exclusive treatment solely with collagenase (i.e., a collagenase digestion or a collagenase decomposition, respectively) for a partial collagenase digestion, wherein *Clostridium histolyticum* collagenase is used in a concentration of 200 CDU to 250 CDU per mL at approximately 36°C to 37°C for a period of approximately 4 to 5 hours,
(d) the collagenase digestion product obtained in (c) is centrifuged, preferably for about 5 minutes at 50 x ***g*** and room temperature,
(e) the pellet obtained in (d) is separated from the supernatant,
(f) the pellet of the collagenase digestion product from (e) is decomposed and resuspended in culture medium and thereby aspirated and returned again and sucked in again for about 4-6 times by means of an aspiration device, preferably a pipette tip, thereby subjected to a treatment with hydrodynamic shear forces, and next transferred in a culture vessel,
(g) the resuspension obtained in (f) is analysed with regard to at least one cell cluster with the help of a microscope (preferably an inverse microscope equipped with a phase lens system), wherein this cell cluster can be identified either on the basis of its phenotypic appearance as a cluster of malignant cells or on the basis of its phenotypic appearance as a cluster of endothelial cells or on the basis of its phenotypic appearance as a cluster of fibroblasts, and
(h) the cell clusters identified in (g) are separated.

2. The method according to Claim 1, **characterized in that** in step (c) the *Clostridium histolyticum* collagenase is used in a concentration of about 230 CDU/mL.

3. The method according to Claim 1 or 2, **characterized in that** in step (g) the resuspension is analysed with regard to cell clusters of malignant cells and also with regard to cell clusters of fibroblasts and/or cell clusters of endothelial cells, and that the cell clusters identified are picked up separately, preferably by means of an aspiration device, and transferred each to a separate culture vessel.

4. The method according to any one of Claims 1 to 3, **characterized in that** in step (g) the resuspension is subjected to a density gradient centrifugation in a separation solution of sucrose polymer, preferably sucrose-epichlorohydrin copolymer (e.g., Ficoll) in culture medium, where the clusters of malignant cells sediment at higher concentrations of the sucrose polymer than the clusters of fibroblast or of endothelial cells.

5. The method according to any one of Claims 1 to 3, **characterized in that** in step (g) the resuspension is transferred to a separation chamber for centrifugal elutriation and then elutriated, wherein the clusters of malignant cells accumulate at higher flow rates than the clusters of fibroblasts or the clusters of endothelial cells.

6. The method according to any one of Claims 1 to 5, **characterized in that** it is a precursor method for a method for testing the sensitivity and possible resistance of the cells of a malignant tumor tissue sample *ex vivo* to a known chemotherapeutic and/or radiotherapeutic agent.

7. The method for testing the response of malignant cells of a malignant tumor tissue sample *ex vivo* to a known chemotherapeutic and/or radiotherapeutic agent, **characterized in that**
the malignant tumor tissue sample is first subjected to a method according to any one of Claims 1 to 5, and only the clusters of malignant cells thereby identified are used in the method for evaluation/testing.

8. The method according to Claim 7, **characterized in that** the method for testing comprises the following measures:
i) transferring the identified clusters of malignant cells to containers with a coating of extracellular matrix components, plating them out on this coating and incubating them with the chemotherapeutic and/or radiotherapeutic agent,
ii) analyzing the number of cells and/or the number of colonies, preferably after performing a cytokeratin staining,
iii) wherein the cell culture medium used in steps i) and ii) contains less than 100 nmol of flavin per 1 litre and is free of phenol red,
iv) and wherein steps i) and ii) are performed in the absence of light of wavelengths below 520 nm,
v) determining the IC50 value for the chemotherapeutic or radiotherapeutic agent or for the combination of chemotherapeutic and radiotherapeutic agent.

9. A method for testing the growth-inhibiting effect of a known chemotherapeutic and/or radiotherapeutic agent on a malignant tumor tissue sample *ex vivo*, whereby the malignant tumor tissue sample is exposed to the chemotherapeutic agent and/or radiotherapeutic agent and then the number of cells and/or cell colonies is analysed and finally the IC50 value of the chemotherapeutic agent or radiotherapeutic agent or the combination of chemotherapeutic and radiotherapeutic agent is determined, and that is **characterized in that** the malignant tumor tissue sample, before being exposed to the chemotherapeutic agent and/or radiotherapeutic agent, is dispersed into stromal cells and malignant cells by a method according to any one of Claims 1 to 5, and only the malignant cells are incubated with the chemotherapeutic agent and/or radiotherapeutic agent and then analysed with regard to the IC50 value.

10. The method according to any one of Claims 3 to 5, **characterized in that** the separated cell clusters of endothelial cells are subjected to a method for the verification of the effect of substances which inhibit angioneogenesis.

## Revendications

1. Procédé pour séparer des amas de cellules malignes et des amas de cellules stromales d'un échantillon de tissu tumoral malin *ex vivo,* **caractérisé en ce que** la manière et l'ordre des étapes suivantes:
(a) l'échantillon de tissu tumoral malin est purifié et haché, de préférence en morceaux d'environ 1-4 mm³ (millimètre cube) de volume,
(b) le tissu haché est mis en suspension dans un milieu de culture,
(c) la suspension obtenue en (b) est soumise à un traitement exclusif avec uniquement de la collagénase (c'est-à-dire une digestion par la collagénase ou une décomposition par la collagénase) pour une digestion partielle par la collagénase, dans lequel de la collagénase de Clostridium histolyticum est utilisée dans une concentration comprise entre 200 CDU et 250 CDU par ml à environ 36 °C à 37 °C durant environ 4 à 5 heures,
(d) le produit de digestion par la collagénase obtenu en (c) est centrifugé, de préférence pendant environ 5 minutes à 50 x g et à température ambiante,
(e) le granulé obtenu en (d) est séparé du liquide surnageant,
(f) le granulé de la digestion par la collagénase obtenu en (e) est décomposé et remis en suspension dans un milieu de culture et à cette occasion il est prélevé et rendu puis réaspiré environ 4-6 fois au moyen d'un instrument d'aspiration, de préférence un embout de pipette, et il est ainsi soumis à un traitement par des forces de cisaillement hydrodynamiques, et il est transféré ensuite dans un récipient de culture,
(g) la remise en suspension obtenue en (f) est analysée à l'aide d'un microscope (de préférence un microscope inversé équipé d'un système de lentilles de phase) en ce qui concerne au moins un amas de cellules qui peut être identifié soit comme étant un amas de cellules malignes sur la base de son apparence phénotypique, soit comme étant un amas de cellules endothéliales sur la base de son apparence phénotypique, soit comme étant un amas de fibroblastes sur la base de son apparence phénotypique, et
(h) les amas de cellules identifiés en (g) sont séparés.

2. Procédé selon la revendication 1, **caractérisé en ce qu'**à l'étape (c), la collagénase *histolyticum Clostridium* est utilisée à une concentration d'environ 230 CDU/ml.

3. Procédé selon la revendication 1 ou 2, **caractérisé en ce qu'**à l'étape (g), la remise en suspension est analysée aussi bien en ce qui concerne les amas cellulaires de cellules malignes et qu'en ce qui concerne les amas cellulaires de cellules de fibroblastes et/ou les amas cellulaires de cellules endothéliales, et **en ce que** les amas cellulaires identifiés sont prélevés séparément, de préférence au moyen d'un dispositif d'aspiration, et sont transférés chacun dans un récipient de culture distinct.

4. Procédé selon l'une des revendications 1 à 3, **caractérisé en ce qu'**à l'étape (g), la remise en suspension est soumise à une centrifugation par gradient de densité dans une solution de séparation à base de polymère de saccharose, de préférence un copolymère de saccharose et d'épichlorhydrine (par exemple, Ficoll) dans un milieu de culture, les amas de cellules malignes sédimentant à des concentrations plus élevées du polymère de saccharose que les amas de fibroblastes ou de cellules endothéliales.

5. Procédé selon l'une des revendications 1 à 3, **caractérisé en ce qu'**à l'étape (g), la remise en suspension est transférée dans une chambre de séparation pour élutriation centrifuge, puis elle est élutriée, les amas de cellules malignes s'accumulant à des débits plus élevés que les amas de fibroblastes ou les amas de cellules endothéliales.

6. Procédé selon l'une des revendications 1 à 5, **caractérisé en ce qu'**il s'agit d'un procédé préalable d'un procédé pour tester la sensibilité et la résistance éventuelle de cellules d'un échantillon de tissu tumoral malin *ex vivo* à un agent de chimiothérapie et/ou à un agent de radiothérapie connu.

7. Méthode pour tester la réponse de cellules malignes d'un échantillon de tissu tumoral malin *ex vivo* à un agent de chimiothérapie et/ou un agent de radiothérapie connu, **caractérisé en ce que**
l'échantillon de tissu tumoral malin est d'abord soumis à un procédé selon l'une des revendications 1 à 5, et que seuls les amas de cellules malignes ainsi identifiés sont utilisés dans le procédé d'évaluation/de test.

8. Procédé selon la revendication 7, **caractérisé en ce que** le procédé de test comprend les mesures suivantes:
i) transfert des amas identifiés de cellules malignes dans des récipients munis d'un revêtement de composants de matrice extracellulaire, étalement sur ce revêtement et incubation avec l'agent de chimiothérapie et/ou l'agent de radiothérapie,
ii) analyse du nombre de cellules et/ou du nombre de colonies, de préférence après avoir effectué une coloration cytokératine,
iii) le milieu de culture cellulaire utilisé dans les étapes i) et ii) contenant moins de 100 nmol par litre de flavine et étant exempt de rouge de phénol,
iv) les étapes i) et ii) étant réalisées en absence de lumière de longueur d'onde inférieure à 520 nm,
v) détermination de la valeur CI50 pour l'agent de chimiothérapie ou de radiothérapie ou pour la combinaison de l'agent de chimiothérapie et de l'agent de radiothérapie.

9. Procédé pour tester l'effet d'inhibition de croissance d'un agent de chimiothérapie et/ou d'un agent de radiothérapie connu sur un échantillon de tissu tumoral malin *ex vivo,* dans lequel l'agent de chimiothérapie et/ou l'agent de radiothérapie est incubé avec l'échantillon de tissu tumoral malin, puis le nombre de cellules et/ou de colonies de cellules sont analysées et enfin la valeur CI50 de l'agent de chimiothérapie ou de l'agent de radiothérapie ou de la combinaison de l'agent de chimiothérapie et de l'agent de radiothérapie est déterminée, et qui est caractérisé ce que, avant l'incubation avec l'agent de chimiothérapie et/ou l'agent de radiothérapie, l'échantillon de tissu tumoral malin est décomposé en des cellules stromales et en des cellules malignes par un procédé selon l'une des revendications 1 à 5, et en ce que seules les cellules malignes sont incubées avec l'agent de chimiothérapie et/ou l'agent de radiothérapie puis analysées du point de vue de leur valeur IC50.

10. Procédé selon l'une des revendications 3 à 5, **caractérisé en ce que** les amas cellulaires de cellules endothéliales séparés sont soumis à un procédé pour tester l'effet de substances inhibant la néogenèse vasculaire.
